# EUROPÄISCHE PATENTSCHRIFT

(11) **EP 0 489 788 B1**
(45) Veröffentlichungstag und Bekanntmachung des Hinweises auf die Patenterteilung: **28.09.1994**
(21) Anmeldenummer: 90912850.6
(22) Anmeldetag: 22.08.1990
(51) Int. Cl.: C07C 317/22, C07C 315/00

(54) **VERFAHREN ZUR HERSTELLUNG VON 4,4'-DIHYDROXYDIPHENYLSULFON**
METHOD OF PREPARATION OF 4,4'-DIHYDROXYDIPHENYLSULPHONE
PROCEDE DE PRODUCTION DE 4,4'-DIHYDROXYDIPHENYLSULFONE

(30) Priorität: 30.08.1989 DE 3928704
(43) Veröffentlichungstag der Anmeldung: 17.06.1992
(73) Patentinhaber: HOECHST AKTIENGESELLSCHAFT, 65926 Frankfurt am Main (DE)
(72) Erfinder: GRÖTSCH, Georg, D-6238 Hofheim am Taunus (DE)
(86) Internationale Anmeldenummer: EP9001388
(87) Internationale Veröffentlichungsnummer: WO9103462

(56) Entgegenhaltungen:
- DE-A- 2 708 388

## Beschreibung

Die Erfindung betrifft ein verbessertes verfahren zur Herstellung von 4,4'-Dihydroxydiphenylsulfon in hoher Ausbeute und in hoher Reinheit durch Umsetzung von Phenol mit einem Sulfonierungsmittel in einem Chlortoluol.

4,4'-Dihydroxydiphenylsulfon ist eine wertvolle Ausgangsverbindung für die Herstellung von Hochleistungskunststoffen, beispielsweise Polyestern, Polyethersulfonen, Epoxyharzen, Polycarbonaten oder Polyurethanen.

Bei einem Großteil der bekannten verfahren zur Herstellung von 4,4'-Dihydroxydiphenylsulfon in hoher Reinheit und Ausbeute (> 90 %) aus Phenol und einem Sulfonierungsmittel unter Auskreisen des dabei gebildeten Wassers (DE-OS 3 723 401, WO 86/6370, JP 86/36253, DE-PS 2 708 388) wird das Reaktionsgemisch während bzw. nach Abschluß der Umsetzung und der Isomerisierung von 2,4'-Dihydroxydiphenylsulfon zum 4,4'-Dihydroxydiphenylsulfon im Vakuum bis zur Trockene eingeengt. Durch diese Maßnahme sind diese bekannten Verfahren für eine Durchführung im technischen Maßstab äußerst ungeeignet.

Gemäß DE-OS 3 723 401 werden Molybdän- und Wolframsäure bzw. Heteropolysäuren als Kondensationskatalysatoren eingesetzt, die nach Beendigung der Umsetzung aus der Reaktionsmischung z.B. durch Filtration entfernt werden. Anschließend wird die phenolische Mutterlauge bis zur Trockene eingeengt. Aufgrund dieses Vorgehens kann eine Kontamination des Dihydroxydiphenylsulfons mit Schwermetallen nicht ausgeschlossen werden, so daß die Qualität der daraus hergestellten Kunststoffe beeinträchtigt werden kann.

Nach WO 86/6370 verbleiben die als Katalysatoren eingesetzten aromatischen Sulfonsäuren bei der beschriebenen Aufarbeitung aufgrund ihrer Schwerflüchtigkeit im Reaktionsprodukt. Eine Verunreinigung des Reaktionsproduktes mit diesen Verbindungen kann daher nicht sicher ausgeschlossen werden.

Das in DE-PS 2 804 080 beschriebene Verfahren (Umsetzung von Phenol mit SO₃ in flüssigem Fluorwasserstoff) erfordert aufgrund der Anwendung von Fluorwasserstoff eine aufwendige, teure apparative Ausstattung und ist deshalb ökonomisch nicht vorteilhaft.

Gemäß EP-OS 0 220 004 wird 4,4'-Dihydroxydiphenylsulfon mit einem Reingehalt von 93-95 % nach einem Verfahren hergestellt, das den weiter oben genannten Nachteil einer Destillation bis zur Trockene vermeidet. Bei der Umsetzung von Phenol mit Schwefelsäure in Gegenwart eines Di- bzw. Trichlorbenzols oder einer Mischung dieser Solventien kristallisiert 4,4'-Dihydroxydiphenylsulfon weitgehend selektiv aus der Reaktionsmischung aus. Die Ausbeute ist jedoch mit maximal 82 % unbefriedigend. Ein großer Anteil des eingesetzten bzw. gebildeten Materials verbleibt im Lösungsmittel und muß aufwendig entsorgt werden.

Nach EP-OS 0 293 037 wird 4,4'-Dihydroxydiphenylsulfon durch Umsetzung von Phenol mit Schwefelsäure in einem Suspendierungsmittel (Isoparaffin) und einem mit Wasser ein Azeotrop bildenden, anderen Solvens (ebenfalls ein Isoparaffin) erhalten. Die mit HPLC im Reaktionsgemisch bestimmte Ausbeute beträgt maximal 96 %. Die dort angegebene Reaktionszeit beträgt allerdings 16 h, wodurch die Wirtschaftlichkeit des Verfahrens stark vermindert wird; eine isolierte Ausbeute wird nicht genannt.

Es bestand somit nach wie vor ein Bedürfnis für ein vorteilhaftes, technisch problemlos realisierbares Verfahren zur Herstellung von 4,4'-Dihydroxydiphenylsulfon in hoher Reinheit bei gleichzeitig hoher Ausbeute.

Es wurde nun überraschenderweise gefunden, daß man 4,4'-Dihydroxydiphenylsulfon in hoher Ausbeute und hoher Reinheit durch Umsetzung von Phenol mit einem Sulfonierungsmittel in einem chlorierten Aromaten herstellen kann, indem man Phenol mit einem Sulfonierungsmittel in ortho-, meta- oder para-Chlortoluol oder in einem beliebigen Gemisch dieser isomeren Chlortoluole als inertem Lösungsmittel bei Temperaturen von etwa 100 bis etwa 200°C, vorzugsweise von etwa 110 bis etwa 170°C, umsetzt und das sich aus dem Reaktionssystem abscheidende 4,4'-Dihydroxydiphenylsulfon, gegebenenfalls nach Zugabe von Wasser und Entfernung des Lösungsmittels, abtrennt.

Als Sulfonierungsmittel sind beispielsweise folgende geeignet: In erster Linie etwa 20 bis etwa 80%iges, vorzugsweise etwa 40 bis etwa 70%iges Oleum; ferner etwa 70 bis etwa 100%ige Schwefelsäure in Gegenwart einer Perfluoralkansulfonsäure, wie beispielsweise Trifluormethan- oder Hexafluorpropansulfonsäure (in einer Menge von etwa 0,01 bis etwa 10 Mol-%, bezogen auf die eingesetzte Schwefelsäure als Sulfonierungsmittel), sowie die anderen bekannten Sulfonierungsmittel, wie beispielsweise Chlorsulfonsäure, die sich intermediär bildende Phenolsulfonsäure oder Schwefelsäure in Gegenwart aromatischer Sulfonsäuren.

Beim erfindungsgemäßen Verfahren wird in der Regel und zweckmäßigerweise 1 Mol Phenol mit einer Menge an Sulfonierungsmittel umgesetzt, die etwa 0,80 bis etwa 0,25, vorzugsweise etwa 0,6 bis etwa 0,4 Moläquivalenten SO₃ entspricht. Sofern im Rahmen der vorstehend als zweckmäßig angegebenen Mengenbereiche 1 Mol Phenol mit einem Sulfonierungsmittel in einer Menge von weniger als 0,5 Moläquivalenten SO₃ umgesetzt wird, so bezieht sich die Ausbeute an 4,4'-Dihydroxydiphenylsulfon in Prozent der Theorie auf das im Unterschuß eingesetzte Sulfonierungsmittel.

Die als inerte Lösungsmittel (Reaktionsmedium) dienenden Chlortoluole bzw. deren Mischungen werden in der Regel in einer Menge von etwa 50 bis etwa 200 Gew.-%, vorzugsweise etwa 70 bis etwa 130 Gew.-%, bezogen auf die theoretisch zu erwartende Menge an 4,4'-Dihydroxydiphenylsulfon eingesetzt.

Das gebildete Reaktionswasser wird kontinuierlich mit Chlortoluol ausgekreist, das Solvens nach Phasentrennung in einem Wasserabscheider ständig in das Reaktionsgemisch zurückgeführt. Es ist aber auch möglich, Chlortoluol in dem Maße zu ergänzen, in dem es aus dem Reaktionsgemisch entfernt wird.

Das 4,4'-Dihydroxydiphenylsulfon wird nach dem erfindungsgemäßen Verfahren in kristalliner Form erhalten und kann direkt aus dem Reaktionsgemisch abgetrennt, beispielsweise abfiltriert, werden.

Die Isolierung ist jedoch auch dadurch möglich, daß man das inerte Lösungsmittel mittels einer Wasserdampfdestillation oder einer azeotropen Destillation unter Wasserzugabe entfernt. Das 4,4'-Dihydroxydiphenylsulfon wird auf diese Weise suspendiert in Wasser erhalten und kann dann ebenfalls durch Filtration isoliert werden.

Das erfindungsgemäße Verfahren kann nicht nur bei Normaldruck, sondern auch bei Über- oder Unterdruck (Vakuum) durchgeführt werden.

Das erfindungsgemäße Verfahren weist gegenüber den bekannten Herstellungsmethoden folgende Vorteile auf:
Es werden keine Schwermetallverbindungen als Katalysatoren eingesetzt, so daß eine entsprechende Kontamination des Produktes bzw. des Abwassers mit Sicherheit ausgeschlossen wird, was u.a. aus ökologischen Gründen notwendig ist.

Das Reaktionsgemisch muß nicht zur Trockne eingeengt werden, um 4,4'-Dihydroxydiphenylsulfon in hoher Reinheit zu erhalten. Dadurch werden alle Probleme vermieden, die mit einem Erstarren des Destillationssumpfes im Reaktionsgefäß zusammenhängen, wie beispielsweise die Notwendigkeit eines erneuten Lösens oder eines Aufschmelzens bzw. das mechanische Austragen von Festprodukt aus dem Reaktionsgefäß. Dies bedeutet einen geringeren apparativen Aufwand und damit geringere Kosten. Außerdem verbleibt der eingesetzte Katalysator bei einer Aufarbeitung in der beschriebenen Art nicht im Produkt, wie dies bei einem Einengen zur Trockne der Fall wäre.

Gegenüber dem Vorgehen nach EP 0 220 004 (Ausbeute nach Beispiel 2: 82 %, bezogen auf Phenol) ist die Ausbeute bei vergleichbarer Reinheit deutlich verbessert (95 %, bezogen auf Phenol).

Der Einsatz Von Chlortoluol an Stelle von Chlorbenzol oder Dichlorbenzolen führt zu einer deutlichen Verbesserung der Reinheit des Produktes um mehr als 10 % (vgl. Beispiele 3 und 4). Dies ist für den Fachmann insofern überraschend, als Chlortoluol bisher nur bei solchen Verfahren als Solvens beschrieben wurde, bei denen die Reaktionsmischung zur Trockene eingeengt wird (WO 86/6370, Beispiel 5; DE-PS 2 708 388, kein Beispiel). Danach mußte man davon ausgehen, daß Chlortoluol bei der Herstellung von möglichst reinem 4,4'-Dihydroxydiphenylsulfon den anderen gebräuchlichen Lösungsmitteln nicht überlegen ist. Die Reaktionszeiten sind gegenüber dem Arbeiten in einem Isoparaffin-Gemisch auf etwa die Hälfte verkürzt, wodurch die Wirtschaftlichkeit erhöht wird. Das Chlortoluol dient gleichzeitig zum azeotropen Wasserauskreisen und als inertes Lösungsmittel, so daß kein Lösungsmittel-Gemisch eingesetzt werden muß. Außerdem werden in der EP-OS 0 293 037 keine isolierten Ausbeuten genannt. Ein Vergleich von Reinheit und Ausbeute der isolierten Ware mit den HPLC-Ausbeuten ist kaum möglich.

Durch die nachstehenden Beispiele wird die Erfindung näher erläutert, ohne darauf beschränkt zu werden.

### Beispiel 1

Zu 188 g geschmolzenem Phenol wurden 89 g 65%iges Oleum zugegeben, wobei etwa 120°C erreicht wurden. Nach Zusatz von 225 g o-Chlortoluol wurden insgesamt 23 g Wasser unter Steigerung der Reaktionstemperatur auf etwa 150°C in etwa 9 h ausgekreist. (Wenn annähernd die Hälfte dieser theoretisch zu erwartenden Wassermenge entfernt ist, beginnt 4,4'-Dihydroxydiphenylsulfon auszukristallisieren.) Nach Beendigung der Umsetzung wurde Wasser zugegeben und o-Chlortoluol in einer azeotropen Destillation entfernt. Das nun in Wasser suspendierte Kristallisat wurde abgesaugt, mit Wasser gewaschen und getrocknet. Gewonnen wurden 237 g Reaktionsprodukt, was einer Ausbeute von 95 % der Theorie, bezogen auf Phenol, entspricht. Der Gehalt an 4,4'-Dihydroxydiphenylsulfon (HPLC) betrug 93 Flächenprozent.

### Beispiel 2

Analog Beispiel 1 wurden 188 g Phenol mit 89 g 65%igem Oleum in Gegenwart von 225 g p-Chlortoluol umgesetzt. Gewonnen wurden 232 g Reaktionsprodukt, was einer Ausbeute von 93 % der Theorie, bezogen auf Phenol, entspricht. Der Gehalt an 4,4'-Dihydroxydiphenylsulfon (HPLC) betrug 94 Flächenprozent.

### Beispiel 3 (Vergleichsbeispiel unter Verwendung von Chlorbenzol an Stelle von Chlortoluol)

Analog Beispiel 1 wurden 188 g Phenol mit 89 g 65%igem Oleum in Gegenwart von 208 g Chlorbenzol umgesetzt. Erhalten wurden 201 g Reaktionsprodukt, was einer Ausbeute von 80 % der Theorie, bezogen auf Phenol, entspricht. Der Gehalt an 4,4'-Dihydroxydiphenylsulfon (HPLC) betrug 82 Flächenprozent.

### Beispiel 4 (Vergleichsbeispiel unter Verwendung von o-Dichlorbenzol an Stelle von Chlortoluol)

Analog Beispiel 1 wurden 188 g Phenol mit 89 g 65%igem Oleum in Gegenwart von 272 g o-Dichlorbenzol umgesetzt. Nach Ende der Reaktion wurde das gebildete 4,4'-Dihydroxydiphenylsulfon direkt aus der Reaktionsmischung abgesaugt. Erhalten wurden 234 g Reaktionsprodukt, was einer Ausbeute von 93 % der Theorie, bezogen auf Phenol, entspricht. Der Gehalt an 4,4'-Dihydroxydiphenylsulfon (HPLC) betrug 79 Flächenprozent.

### Beispiel 5

Bei 130°C wurden 102 g 96 %ige Schwefelsäure zu 188 g Phenol zugetropft. Anschließend wurden 225 g o-Chlortoluol und 4,6 g 2-Hydrohexafluorpropansulfonsäure zugegeben. Unter Steigerung der Reaktionstemperatur auf 150°C wurde Wasser ausgekreist. (Wenn etwa die Hälfte der theoretisch zu erwartenden Wassermenge von 40 g abdestilliert ist, beginnt 4,4'-Dihydroxydiphenylsulfon auszukristallisieren.) Nach Reaktionsende (40 g Wasser wurden abgetrennt; die Reaktionszeit betrug etwa 7 Stunden) wurde unter Zugabe von 900 g Wasser o-Chlortoluol quantitativ in azeotroper Destillation entfernt, wobei zugleich 260 g Wasser mit übergetrieben wurden. Nach Abkühlen auf Raumtemperatur wurde der Niederschlag abgesaugt, mit Wasser gewaschen und getrocknet.

Erhalten wurden 233 g Reaktionsprodukt, was einer Ausbeute von 93 % der Theorie, bezogen auf Phenol, entspricht. Der Gehalt an 4,4'-Dihydroxydiphenylsulfon (HPLC) betrug 93 Flächenprozent.

### Beispiel 6

Analog Beispiel 1 wurden 188 g Phenol mit 89 g 65%igem Oleum in Gegenwart von 230 g m-Chlortoluol umgesetzt. Erhalten wurden 234 g Reaktionsprodukt, was einer Ausbeute von 94 % der Theorie, bezogen auf Phenol, entspricht. Der Gehalt an 4,4'-Dihydroxydiphenylsulfon (HPLC) betrug 93 Flächenprozent.

### Beispiel 7

Analog Beispiel 1 wurden 188 g Phenol mit 89 g 65%igem Oleum in Gegenwart von 230 g Chlortoluol (Zusammensetzung: 49 % o-, 1 % m- und 50 % p-Isomer) umgesetzt. Ausbeute und Reinheit an 4,4'-Dihydroxydiphenylsulfon entsprechen Beispiel 1.

## Patentansprüche

1. Verfahren zur Herstellung von 4,4'-Dihydroxydiphenylsulfon in hoher Ausbeute und hoher Reinheit durch Umsetzung von Phenol mit einem Sulfonierungsmittel in einem chlorierten Aromaten, dadurch gekennzeichnet, daß man Phenol mit einem Sulfonierungsmittel in ortho-, meta- oder para-Chlortoluol oder in einem beliebigen Gemisch dieser isomeren Chlortoluole als inertem Lösungsmittel bei Temperaturen von etwa 100 bis etwa 200°C umsetzt und das sich aus dem Reaktionssystem abscheidende 4,4'-Dihydroxydiphenylsulfon, gegebenenfalls nach Zugabe von Wasser und Entfernung des Lösungsmittels, abtrennt.

2. Verfahren nach Anspruch 1, dadurch gekennzeichnet, daß man bei Temperaturen von etwa 110 bis etwa 170°C umsetzt.

3. Verfahren nach mindestens einem der Ansprüche 1 und 2, dadurch gekennzeichnet, daß man die Chlortoluole oder deren Mischungen in einer Menge von etwa 50 bis etwa 200 Gew.-%, bezogen auf die theoretisch zu erwartende Menge an 4,4'-Dihydroxydiphenylsulfon, einsetzt.

4. Verfahren nach mindestens einem der Ansprüche 1 bis 3, dadurch gekennzeichnet, daß man die Chlortoluole oder deren Mischungen in einer Menge von etwa 70 bis etwa 130 Gew.-%, bezogen auf die theoretisch zu erwartende Menge an 4,4'-Dihydroxydiphenylsulfon, einsetzt.

5. Verfahren nach mindestens einem der Ansprüche 1 bis 4, dadurch gekennzeichnet, daß man als Sulfonierungsmittel etwa 20 bis 80%iges Oleum, etwa 70 bis 100%ige Schwefelsäure, Chlorsulfonsäure oder Phenolsulfonsäure einsetzt.

6. Verfahren nach mindestens einem der Ansprüche 1 bis 5, dadurch gekennzeichnet, daß man 1 Mol Phenol mit einer Menge an Sulfonierungsmittel umsetzt, die etwa 0,80 bis etwa 0,25 Moläquivalenten SO₃ entspricht.

7. Verfahren nach mindestens einem der Ansprüche 1 bis 6, dadurch gekennzeichnet, daß man 1 Mol Phenol mit einer Menge an Sulfonierungsmittel umsetzt, die etwa 0,6 bis etwa 0,4 Moläquivalenten SO₃ entspricht.

8. Verfahren nach mindestens einem der Ansprüche 1 bis 7, dadurch gekennzeichnet, daß die Umsetzung bei Normaldruck, Überdruck oder Unterdruck (Vakuum) vorgenommen wird.

## Claims

1. A process for the preparation of 4,4'-dihydroxydiphenyl sulfone in a high yield and high purity by reaction of phenol with a sulfonating agent in a chlorinated aromatic, which comprises reacting phenol with a sulfonating agent in ortho-, meta- or para-chlorotoluene or in any desired mixture of these isomeric chlorotoluenes as the inert solvent, at a temperature of from about 100 to about 200°C and isolating the 4,4'-dihydroxydiphenyl sulfone which separates out of the reaction system, if appropriate after adding water and removing the solvent.

2. The process as claimed in claim 1, wherein the reaction is carried out at a temperature of from about 110 to about 170°C.

3. The process as claimed in at least one of claims 1 and 2, wherein the chlorotoluene or mixture of chlorotoluenes is employed in an amount of from about 50 to about 200% by weight, based on the amount of 4,4'-dihydroxydiphenyl sulfone to be expected in theory.

4. The process as claimed in at least one of claims 1 to 3, wherein the chlorotoluene or mixture of chlorotoluenes is employed in an amount of from about 70 to about 130% by weight, based on the amount of 4,4'-dihydroxydiphenyl sulfone to be expected in theory.

5. The process as claimed in at least one of claims 1 to 4, wherein from about 20 to about 80% strength oleum, from about 70 to 100% strength sulfuric acid, chlorosulfonic acid or phenolsulfonic acid is employed as the sulfonating agent.

6. The process as claimed in at least one of claims 1 to 5, wherein 1 mole of phenol is reacted with an amount of sulfonating agent which corresponds to from about 0.80 to about 0.25 mole equivalent of SO₃.

7. The process as claimed in at least one of claims 1 to 6, wherein 1 mole of phenol is reacted with an amount of sulfonating agent which corresponds to from about 0.6 to about 0.4 mole equivalent of SO₃.

8. The process as claimed in at least one of claims 1 to 7, wherein the reaction is carried out under normal pressure, increased pressure or reduced pressure (vacuum).

## Revendications

1. Procédé pour préparer la 4,4'-dihydroxydiphénylsulfone avec un rendement élevé et une pureté élevée, en faisant réagir le phénol avec un agent de sulfonation dans un composé aromatique chloré, caractérisé en ce que l'on fait réagir le phénol avec un agent de sulfonation dans de l'ortho-, méta- ou para-chlorotoluène, ou dans un mélange quelconque de ces chlorotoluènes isomères comme solvants inertes, à des températures d'environ 100 à environ 200°C, et en ce que l'on sépare la 4,4'-dihydroxydiphénylsulfone qui précipite du système réactionnel, éventuellement après l'addition d'eau et l'élimination du solvant.

2. Procédé selon la revendication 1, caractérisé en ce que l'on effectue la réaction à des températures d'environ 110 à environ 170°C.

3. Procédé selon au moins une des revendications 1 et 2, caractérisé en ce que l'on utilise les chlorotoluènes ou leurs mélanges dans une quantité d'environ 50 à environ 200 % en poids, calculée sur la quantité,prévue par la théorie, de 4,4'-dihydroxydiphénylsulfone.

4. Procédé selon au moins une des revendications 1 à 3, caractérisé en ce que les chlorotoluènes ou leurs mélanges sont utilisés dans une quantité d'environ 70 à environ 130 % en poids, calculée sur la quantité, prévue par la théorie, de 4,4'-dihydroxydiphénylsulfone.

5. Procédé selon au moins une des revendications 1 à 4, caractérisé en ce que, comme agent de sulfonation, on utilise l'oléum à environ 20-80 %, l'acide sulfurique à environ 70-100 %, l'acide chlorosulfonique ou l'acide phénolsulfonique.

6. Procédé selon au moins une des revendications 1 à 5, caractérisé en ce que l'on fait réagir une mole de phénol avec une quantité d'agent de sulfonation qui correspond environ à 0,80-0,25 équivalent molaire de SO₃.

7. Procédé selon au moins une des revendications 1 à 6, caractérisé en ce que l'on fait réagir une mole de phénol avec une quantité d'agent de sulfonation qui correspond d'environ 0,6 à environ 0,4 équivalent molaire de SO₃.

8. Procédé selon au moins une des revendications 1 à 7, caractérisé en ce que la réaction est effectuée à la pression normale, à une pression supérieure à la normale ou à une pression inférieure à la normale (sous vide).
